# EUROPEAN PATENT APPLICATION

(11) **EP 1 593 678 A1**
(43) Date of publication of application: **09.11.2005**
(21) Application number: 04706350.8
(22) Date of filing: 29.01.2004
(51) Int. Cl.: C07D 307/58, A61K 31/593, A61P 3/10, A61P 3/14, A61P 5/20, A61P 9/12, A61P 11/00, A61P 17/06, A61P 17/10, A61P 17/14, A61P 19/00, A61P 19/08, A61P 19/10, A61P 29/00, A61P 35/00, A61P 43/00, B01D 9/02

(54) **CRYSTAL OF (23S)-1ALPHA-HYDROXY-27-NOR-25-METHYLENEVITAMIN D3-26,23-LACTONE AND PROCESS FOR PRODUCING THE SAME**

(30) Priority: 31.01.2003 JP 2003023365
(71) Applicant: Teijin Pharma Limited, Tokyo 100-0011 (JP)
(72) Inventor: TAKENOUCHI, Kazuya, Teijin Pharma Limited, Hino-shi Tokyo 1910065 (JP); MANABE, Kenji, 1910065 (JP)
(74) Representative: Hallybone, Huw George
(86) International application number: PCT/JP2004/000817
(87) International publication number: WO 2004/067526

(57) **Abstract**

A crystal of (23S)-1α-hydroxy-27-nor-25-methylenevitamin D₃-26,23-lactone described in the following formula is provided which shows a specific powder X-ray diffraction pattern or a specific infrared spectroscopic pattern.

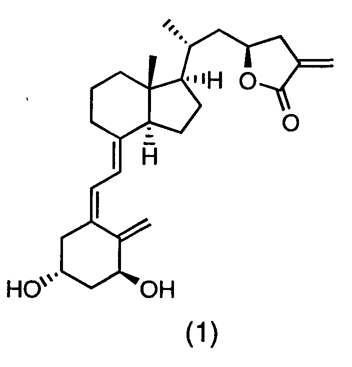

## Description

### TECHNICAL FIELD

The present invention relates to a crystal of (23S)-1α-hydroxy-27-nor-25-methylenevitamin D₃-26,23-lactone, a process for production thereof, and a pharmaceutical composition containing thereof. The compound of the present invention has a binding ability for a vitamin D₃ receptor, and can be used as a therapeutic drug for osteoporosis, malignant tumor, psoriasis, hyperparathyroidism, inflammatory respiratory disease, rheumatoid arthritis, diabetes mellitus, hypertension, alopecia, acne, dermatitis, hypercalcemia, Paget's disease of bone, etc.

### BACKGROUND ART

In the manufacture of a bulk drug substance, it is generally advantageous, with respect to the storage stability, process control in the manufacturing step, etc., to obtain the bulk drug substance in a crystalline state or further in a crystalline state with a specified crystal morphology, compared to obtaining the bulk drug substance in an amorphous form. A compound, (23S)-1α-hydroxy-27-nor-25-methylenevitamin D₃-26,23-lactone, represented by Formula (1) described below is disclosed to have promoting action of bone formation (International Publication WO 95/33716, pamphlet), inhibitory action of neutrophile infiltration (International Publication WO 00/24712, pamphlet), inhibitory action of bone resorption (International Publication WO 02/15894, pamphlet), inhibitory action of parathyroid hormone secretion (International Publication WO 02/17911, pamphlet), etc.

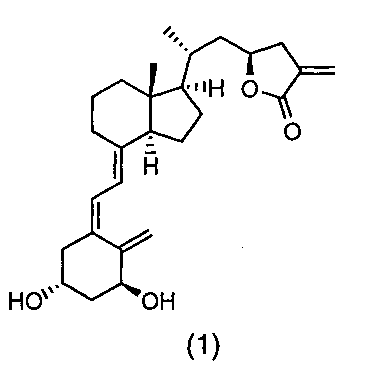

However, in the specifications mentioned above, there is no description about a crystal of the compound, and every form of (23S)-1α-hydroxy-27-nor-25-methylenevitamin D₃-26,23-lactone considered there is an amorphous body.

### DISCLOSURE OF THE INVENTION

An object of the present invention is to provide a crystal of (23S)-1α-hydroxy-27-nor-25-methylenevitamin D₃-26,23-lactone and a process for production thereof.

Also, an object of the present invention is to provide a pharmaceutical composition that contains a crystal of (23S)-1α-hydroxy-27-nor-25-methylenevitamin D₃-26,23-lactone as an active ingredient.

Inventors of the present invention have studied with the object mentioned above, and found that a crystal of the compound can be obtained by performing crystallization from an organic solvent or from a mixed solvent of an organic solvent and water, leading to the present invention.

That is, the present invention can be summarized in the following (1) to (14).
(1) A crystal of (23S)-1α-hydroxy-27-nor-25-methylenevitamin D₃-26,23-lactone which shows characteristic peaks of a powder X-ray diffraction pattern approximately at 12.65°, 15.05°, 15.20°, 16.85°, 17.30°, 17.50°, 18.70°, and 19.10°, whereby the reflection angle is expressed with 2θ.
(2) A crystal of (23S)-1α-hydroxy-27-nor-25-methylenevitamin D₃-26,23-lactone which has characteristic peaks at 3482 cm⁻¹, 2946 cm⁻¹, 1744 cm⁻¹, 1663 cm⁻¹, 1433 cm⁻¹, 1364 cm⁻¹, 1281 cm⁻¹, 1144 cm⁻¹, 1038 cm⁻¹, 957 cm⁻¹, 897 cm⁻¹, 816 cm⁻¹, 741 cm⁻¹, 627 cm⁻¹, and 534 cm⁻¹ in an infrared spectroscopic analysis.
(3) The crystal described in (1) or (2) described above, which is obtained by crystallization from a solution that is prepared by dissolving (23S)-1α-hydroxy-27-nor-25-methylenevitamin D₃-26,23-lactone in an organic solvent.
(4) The crystal described in (1) or (2) described above, which is obtained by crystallization from a solution that is prepared by dissolving (23S)-1α-hydroxy-27-nor-25-methylenevitamin D₃-26,23-lactone in a mixed solvent consisting of an organic solvent and water.
(5) The crystal described in (3) or (4) described above, wherein the above-described organic solvent is 1 kind or 2 kinds or more of solvents selected from the group consisting of methanol, ethanol, acetonitrile, methyl acetate, ethyl acetate, isopropyl acetate, isobutyl acetate, methyl formate, ethyl formate, and acetic acid.
(6) A pharmaceutical composition that contains the crystal of (23S)-1α-hydroxy-27-nor-25-methylenevitamin D₃-26,23-lactone described in any one of (1) to (5) described above as an active ingredient.
(7) The pharmaceutical composition described in (6) described above, which is a therapeutic drug for osteoporosis, malignant tumor, psoriasis, hyperparathyroidism, inflammatory respiratory disease, rheumatoid arthritis, diabetes mellitus, hypertension, alopecia, acne, dermatitis, hypercalcemia, or Paget's disease of bone.
(8) A process for production the crystal described in (1) or (2) described above, wherein the process is characterized by crystallization from a solution that is prepared by dissolving (23S)-1α-hydroxy-27-nor-25-methylenevitamin D₃-26,23-lactone in an organic solvent.
(9) A process for producing the crystal described in (1) or (2) described above, wherein the process is characterized by crystallization from a solution that is prepared by dissolving (23S)-1α-hydroxy-27-nor-25-methylenevitamin D₃-26,23-lactone in a mixed solvent consisting of an organic solvent and water.
(10) The process for production described in (8) or (9) described above, wherein the organic solvent described above is 1 kind or 2 kinds or more of solvents selected from the group consisting of methanol, ethanol, acetonitrile, methyl acetate, ethyl acetate, isopropyl acetate, isobutyl acetate, methyl formate, ethyl formate, and acetic acid.
(11) The process for production described in (8) or (9) described above, wherein the organic solvent described above is 1 kind or 2 kinds or more of solvents selected from the group consisting of methanol, ethanol, acetonitrile, and acetic acid.
(12) The process for production described in (8) or (9) described above, wherein the organic solvent described above is acetonitrile.
(13) The process for production described in (8) or (9) described above, wherein the organic solvent described above is methanol.
(14) The process for production described in (8) or (9) described above, wherein the organic solvent described above is ethanol.

### BRIEF DESCRIPTION OF THE DRAWINGS

Figure 1 is a diagram showing an XRD pattern of an amorphous form of (23S)-1α-hydroxy-27-nor-25-methylenevitamin D₃-26,23-lactone;
Figure 2 is a diagram showing an XRD pattern of crystal A crystallized from a solution prepared by dissolving (23S)-1α-hydroxy-27-nor-25-methylenevitamin D₃-26,23-lactone in a mixed solvent of methanol and water;
Figure 3 is a diagram showing an XRD pattern of crystal A crystallized from a solution prepared by dissolving (23S)-1α-hydroxy-27-nor-25-methylenevitamin D₃-26,23-lactone in a mixed solvent of ethanol and water;
Figure 4 is a diagram showing an XRD pattern of crystal A crystallized from a solution prepared by dissolving (23S)-1α-hydroxy-27-nor-25-methylenevitamin D₃-26,23-lactone in a mixed solvent of acetonitrile and water;
Figure 5 is a diagram showing an XRD pattern of crystal A crystallized from a solution prepared by dissolving (23S)-1α-hydroxy-27-nor-25-methylenevitamin D₃-26,23-lactone in acetonitrile;
Figure 6 is a diagram showing an XRD pattern of crystal A crystallized from a solution prepared by dissolving (23S)-1α-hydroxy-27-nor-25-methylenevitamin D₃-26,23-lactone in ethyl formate; and
Figure 7 is a diagram showing an XRD pattern of crystal A crystallized from a solution prepared by dissolving (23S)-1α-hydroxy-27-nor-25-methylenevitamin D₃-26,23-lactone in ethyl acetate.

### BEST MODE FOR CARRYING OUT THE INVENTION

The crystal of the present invention is characterized by showing characteristic peaks of a powder X-ray diffraction (XRD) pattern approximately at 12.65°, 15.05°, 15.20°, 16.85°, 17.30°, 17.50°, 18.70°, and 19.10°, whereby the reflection angle is expressed with 2θ. In the following, this crystal is called crystal A in order to differentiate it from other crystals.

Also, the crystal of the present invention can be produced by crystallization of the compound from a solution that is prepared by dissolving (23S)-1α-hydroxy-27-nor-25-methylenevitamin D₃-26,23-lactone in an organic solvent or a mixed solvent of an organic solvent and water. As the organic solvent, methanol, ethanol, acetonitrile, methyl acetate, ethyl acetate, isopropyl acetate, isobutyl acetate, methyl formate, ethyl formate, acetic acid and the like can be used. These can be used singly or as a mixed solvent of two or more thereof. Among them, preferable solvents include methanol, ethanol, acetonitrile, and acetic acid. Particularly preferable solvents include acetonitrile, methanol and ethanol as a single organic solvent system, and methanol and water, ethanol and water, acetonitrile and water, and acetic acid and water as a mixed solvent system consisting of an organic solvent and water. In the case of the mixed solvent system consisting of an organic solvent and water, the volume ratio between them is preferably in the range of the organic solvent: water from 1:10 to 10:1.

Production of the crystal of the present invention can be carried out in various processes, and a typical example is as follows.

In the case of crystallization from an organic solvent, (23S)-1α-hydroxy-27-nor-25-methylenevitamin D₃-26,23-lactone is dissolved in the organic solvent at temperatures ranging from room temperature to the boiling point of the organic solvent, subsequently the resultant solution is cooled down to temperatures ranging from -20°C to room temperature, and is left standing. The precipitated crystal is then filtered and dried under reduced pressure.

In the case of crystallization from a mixed solvent of an organic solvent and water, (23S)-1α-hydroxy-27-nor-25-methylenevitamin D₃-26,23-lactone is dissolved in the organic solvent at temperatures ranging from room temperature to the boiling point of the organic solvent, and water is added to the resultant solution while cooling the solution in the air. Subsequently, the solution is cooled down to temperatures ranging from -20°C to room temperature, and is left standing, and the precipitated crystal is then filtered and dried under reduced pressure.

Further, in general, even if it is attempted to prepare a pure crystal of a specified crystal morphology, there are cases in which it is not possible to completely eliminate contamination of a crystal of other morphology or an amorphous body. Even in the process for preparing the crystal of (23S)-1α-hydroxy-27-nor-25-methylenevitamin D₃-26,23-lactone of the present invention, the above possibility cannot be completely ruled out. The present invention refers to a substantially pure state of crystal A or a process for production thereof, and hence comprises also crystals containing minute contamination or the process for production thereof. This is because even such crystals can also achieve the effect of the present invention such as the storage stability of the bulk drug substance and the pharmaceutical composition.

The crystal of (23S)-1α-hydroxy-27-nor-25-methylenevitamin D₃-26,23-lactone obtained in this way can take various morphological forms, but crystal A described above is particularly preferable. As will be described in embodiments, this crystal was found to have dramatically improved storage stability compared to an amorphous body. Accordingly, such a crystal is advantageous with regard to the storage stability of a bulk drug substance and a pharmaceutical composition thereof, control of producing steps, etc., and hence is useful in manufacturing of pharmaceutical products.

Further, (23S)-1α-hydroxy-27-nor-25-methylenevitamin D₃-26,23-lactone used in the present invention can be manufactured, for example, by the method disclosed in the description of WO95/33716.

The pharmaceutical composition that contains (23S)-1α-hydroxy-27-nor-25-methylenevitamin D₃-26,23-lactone as the active ingredient has promoting action of bone formation, inhibitory action of neutrophile infiltration, inhibitory action of bone resorption, inhibitory action of parathyroid hormone secretion, etc. Therefore, the pharmaceutical composition that contains (23S)-1α-hydroxy-27-nor-25-methylenevitamin D₃-26,23-lactone as the active ingredient can be used as a therapeutic drug effective for treating osteoporosis, malignant tumor, psoriasis, hyperparathyroidism, inflammatory respiratory disease, rheumatoid arthritis, diabetes mellitus, hypertension, alopecia, acne, dermatitis, hypercalcemia, Paget's disease of bone, etc.

The pharmaceutical composition of the present invention can contain the crystal of the active ingredient that is (23S)-1α-hydroxy-27-nor-25-methylenevitamin D₃-26,23-lactone and carriers indicated in the following. That is, the carriers are: excipients such as lactose, starch, calcium carbonate, crystalline cellulose, silicic acid or the like in the case of tablet, pill, powder, and granule; binders such as carboxymethyl cellulose, methyl cellulose, calcium phosphate, polyvinylpyrrolidone, or the like; disintegrators such as sodium alginate, sodium bicarbonate, sodium lauryl sulfate, stearic acid monoglyceride, or the like; lubricants such as glycerin, or the like; absorbents such as kaolin, colloidal silica, or the like; lubricants such as talc, granular boric acid, or the like. In the case of liquid preparation such as solution, suspension, syrup etc., the carriers are, for example: glycerin esters such as tricaprillin, triacetin, iodinated poppyseed oil fatty acid ester etc.; water; alcohols such as ethanol; oilbased agents such as liquid paraffin, coconut oil, soybean oil, sesame oil, corn oil, etc.; or the like. In the case of preparations for injections such as intravenous, hypodermic, and intramuscular injection, the carriers include physiological saline, propylene glycol, polyethylene glycol, or vegetable oils such as olive oil, injectable organic esters such as ethyl oleate and iodinated poppyseed oil fatty acid ester, or the like. In the case of preparations such as ointment, the carriers include: fatty oils such as castor oil, olive oil, sesame oil, safflower oil, etc.; lanolin; white, yellow or hydrophilic vaseline; wax; higher alcohols such as oleyl alcohol, isostearyl alcohol, octyldodecanol, hexyldecanol, etc.; glycols such as glycerin, diglycerin, ethyleneglycol, propyleneglycol, sorbitol, 1,3-butanediol, or the like.

Effective dosage of the active ingredient of the present invention is usually in the range from 0.001 to 10,000 µg/day, depending on the route of administration, age, sex, and severity of the disease of the patient, and is dosed in the range from 1-3 times daily to 1-3 times a week. It is preferable to prepare the pharmaceutical composition to satisfy these conditions.

### EXAMPLES

In the following, the present invention will be explained in detail by examples. However, the present invention is not limited to these examples.

### <Reference example 1>

### Production of an amorphous body of (23S)-1α-hydroxy-27-nor-25-methylenevitamin D₃-26,23-lactone

By using HPLC, 6.8 g of (23S)-1α-hydroxy-27-nor-25-methylenevitamin D₃-26,23-lactone produced by the process described in the specification of WO95/33716 was preparatively isolated according to the following conditions:
Column: C 18 column with 100 φ × 500 mm
Mobile phase: 65% (MeOH/CH₃CN = 3/5)/H₂O
Flow rate: 300 mL/min
Detection: 265 nm
Sample injection: 6.8 g sample dissolved in 40 mL MeOH injected in 10 aliquots and preparatively separated.

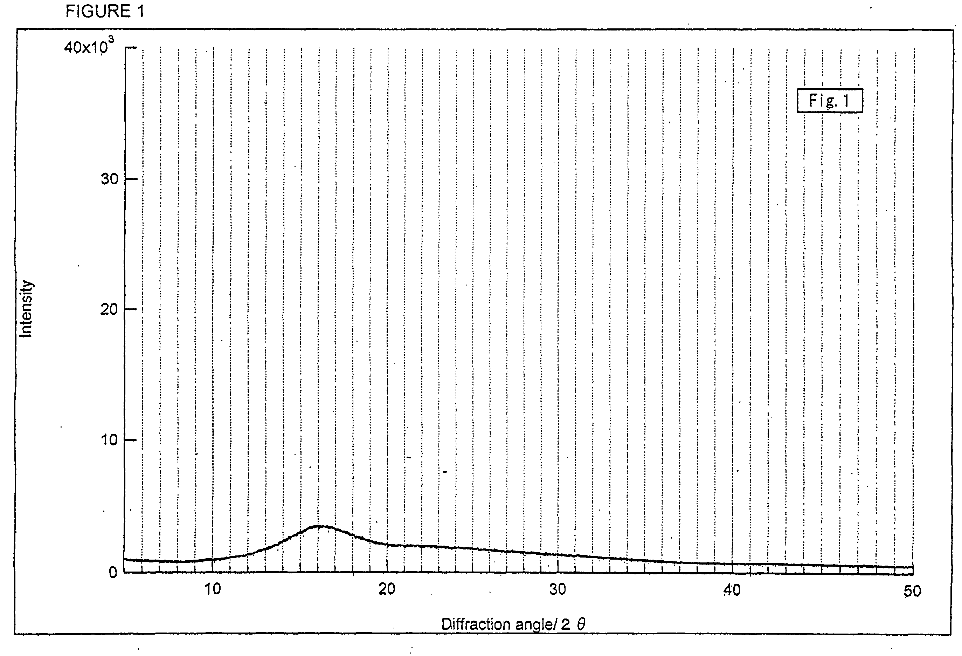

The fraction of the compound was concentrated under reduced pressure. The precipitated solid was filtered out by using a teflon membrane filter (hydrophilic filter with a 2 µm pore size, manufactured by ADVANTEC), and the obtained solid was dried for 12 hours in a freeze-dryer after left standing in a freezer at -30°C for one hour to yield 2.0 g of an amorphous body of the compound. An XRD pattern of this sample is shown in Fig. 1. The measurement of XRD was performed under the following conditions:
Sample preparation: subjected to the measurement by filling the sample on a glass sample table after crushing the sample by using an agate mortar.
Instrument: RIGAKU ROTAFLEX RU300
X-ray source: Cu-Kα (λ = 1.5418 angstrom), 50 kV - 200 mA
Slit: DS 1° - SS 1° - RS 0.15 mm - Graphite
   Monochromator - 0.45 mm
Method: 2θ-θ scan, 0.05 step/sec, scan range 5 - 50°

### <Example 1>

### Production of crystal A of (23S)-1α-hydroxy-27-nor-25-methylenevitamin D₃-26,23-lactone (crystallization from a mixed solvent of methanol and water)

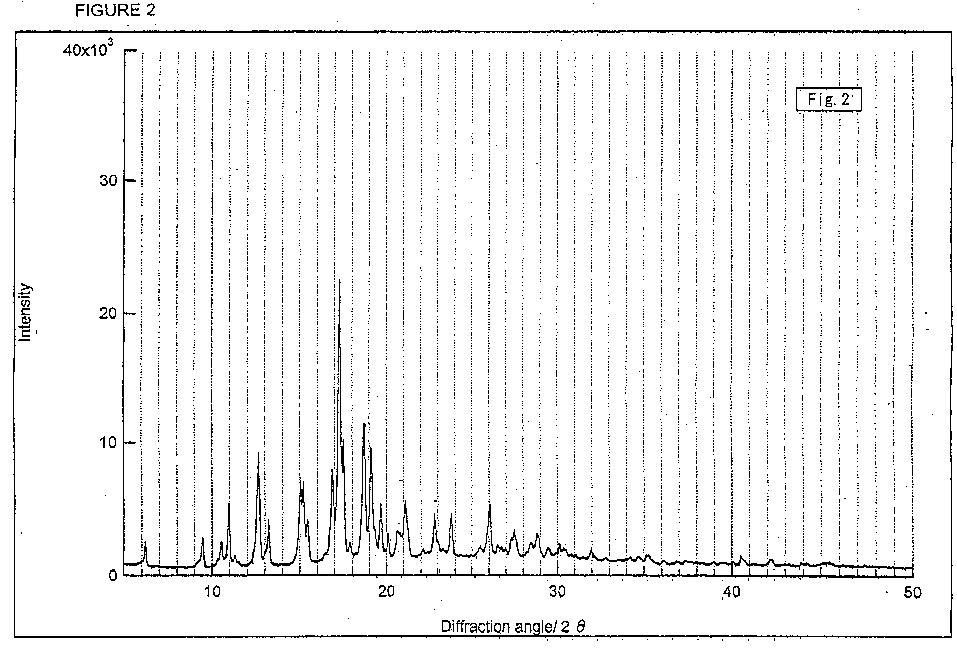

A 200 mg aliquot of (23S)-1α-hydroxy-27-nor-25-methylenevitamin D₃-26,23-lactone was dissolved in 500 µL of methanol at 55°C. After cooling the solution down to room temperature in the air, 120 µL of water was added while stirring gently, and the resultant solution was left standing at room temperature. After precipitation of crystals, the solution was further left standing at 15°C for 2 days. The precipitated crystals were filtered out and dried at room temperature under reduced pressure to yield 138 mg of crystal A of (23S)-1α-hydroxy-27-nor-25-methylenevitamin D₃-26,23-lactone. An XRD pattern of this crystal is shown in Fig. 2. The XRD measurement was performed in the similar manner as described in Reference example 1.

Further, in an infrared spectroscopic analysis of crystal A, characteristic peaks were observed at the following wavenumbers: 3482 cm⁻¹, 2946 cm⁻¹, 1744 cm⁻¹, 1663 cm⁻¹, 1433 cm⁻¹, 1364 cm⁻¹, 1281 cm⁻¹, 1144 cm⁻¹, 1038 cm⁻¹, 957 cm⁻¹, 897 cm⁻¹, 816 cm⁻¹, 741 cm⁻¹, 627 cm⁻¹, and 534 cm⁻¹.

### <Example 2>

### Production of crystal A of (23S)-1α-hydroxy-27-nor-25-methylenevitamin D₃-26,23-lactone (crystallization from a mixed solvent of ethanol and water)

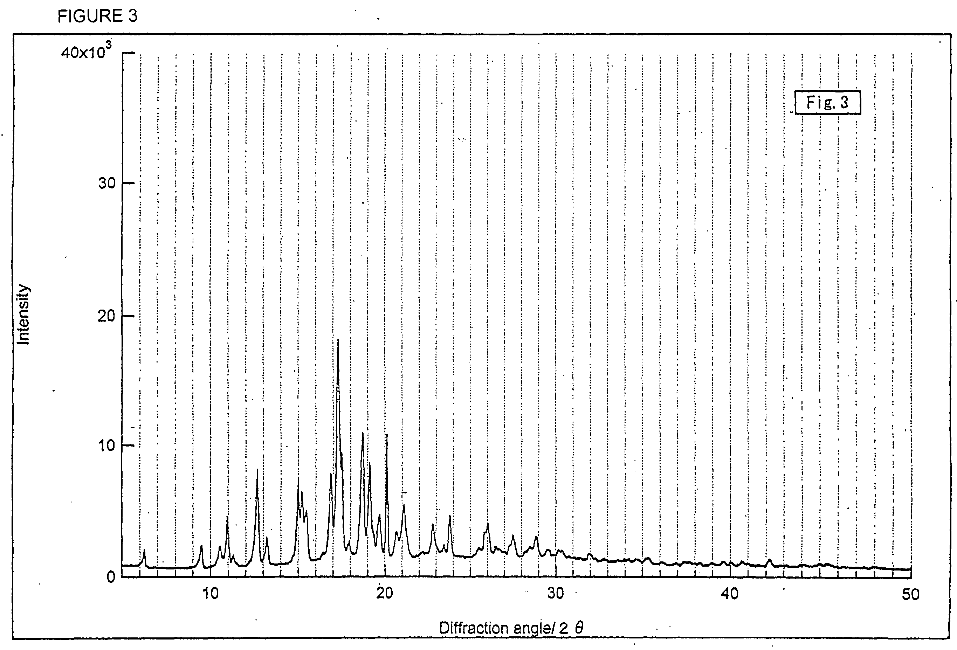

A 200 mg aliquot of (23S)-1α-hydroxy-27-nor-25-methylenevitamin D₃-26,23-lactone was dissolved in 500 µL of ethanol at 60°C. After cooling the solution down to room temperature in the air, 300 µL of water was added while stirring gently, and the resultant solution was left standing at room temperature. After precipitation of crystals, the solution was further left standing at 15°C for 2 days. The precipitated crystals were filtered out and dried at room temperature under reduced pressure to yield 134 mg of crystal A of (23S)-1α-hydroxy-27-nor-25-methylenevitamin D₃-26,23-lactone. An XRD pattern of this crystal is shown in Fig. 3. The XRD measurement was performed in the similar manner as described in Reference example 1.

### <Example 3>

### Production of crystal A of (23S)-1α-hydroxy-27-nor-25-methylenevitamin D₃-26,23-lactone (crystallization from a mixed solvent of acetonitrile and water)

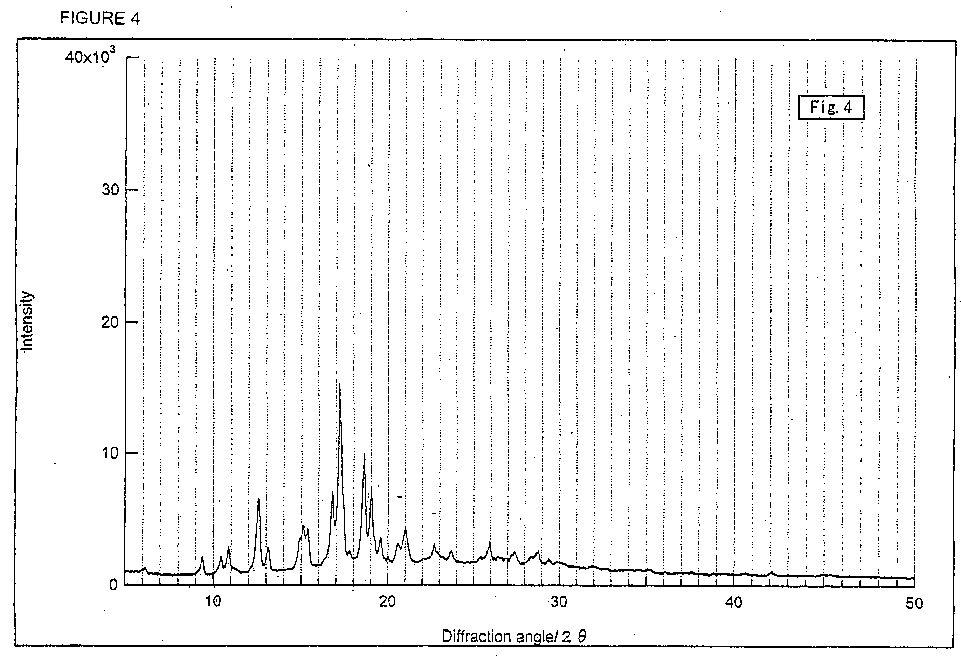

A 200 mg aliquot of (23S)-1α-hydroxy-27-nor-25-methylenevitamin D₃-26,23-lactone was dissolved in 300 µL of acetonitrile while warming. After cooling the solution to 25°C, 45µL of water was added while stirring gently, and the resultant solution was left standing at room temperature. After precipitation of crystals, the solution was further left standing at 20°C for 2 days. The precipitated crystals were filtered out and dried at room temperature under reduced pressure to yield 80 mg of crystal A of (23S)-1α-hydroxy-27-nor-25-methylenevitamin D₃-26,23-lactone. An XRD pattern of this crystal is shown in Fig. 4. The XRD measurement was performed in the similar manner as described in Reference example 1.

### <Example 4>

### Production of crystal A of (23S)-1α-hydroxy-27-nor-25-methylenevitamin D₃-26,23-lactone (crystallization from a mixed solvent of acetic acid and water)

A 200 mg aliquot of (23S)-1α-hydroxy-27-nor-25-methylenevitamin D₃-26,23-lactone was dissolved in 400 µL of acetic acid at 60°C. After cooling the solution down to room temperature in the air, 300 µL of water was added while stirring gently, and the resultant solution was left standing at room temperature. After precipitation of crystals, the solution was further left standing at 15°C for 2 days. The precipitated crystals were filtered out and dried at room temperature under reduced pressure to yield 94 mg of crystal A of (23S)-1α-hydroxy-27-nor-25-methylenevitamin D₃-26,23-lactone.

### <Example 5>

### Production of crystal A of (23S)-1α-hydroxy-27-nor-25-methylenevitamin D₃-26,23-lactone (crystallization from acetonitrile)

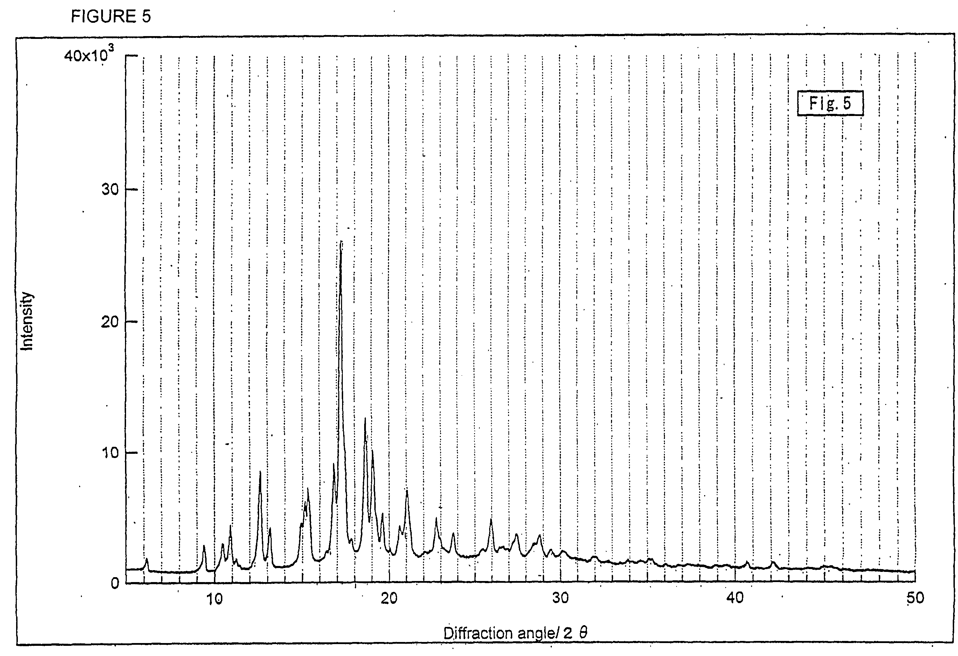

A 200 mg aliquot of (23S)-1α-hydroxy-27-nor-25-methylenevitamin D₃-26,23-lactone was dissolved in 300 µL of acetonitrile while warming. After cooling the solution to 25°C, the solution was left standing. After precipitation of crystals, the solution was further left standing at 20°C for 2 days. The precipitated crystals were filtered out and dried at room temperature under reduced pressure to yield 94 mg of crystal A of (23S)-1α-hydroxy-27-nor-25-methylenevitamin D₃-26,23-lactone. An XRD pattern of this crystal is shown in Fig. 5. The XRD measurement was performed in the similar manner as described in Reference example 1.

### <Example 6>

### Production of crystal A of (23S)-1α-hydroxy-27-nor-25-methylenevitamin D₃-26,23-lactone (crystallization from methyl formate)

A 100 mg aliquot of (23S)-1α-hydroxy-27-nor-25-methylenevitamin D₃-26,23-lactone was dissolved in 150 µL of methyl formate while warming. After cooling the solution to 25°C, the solution was left standing. After precipitation of crystals, the solution was further left standing at 20°C for 2 days. The precipitated crystals were filtered out and dried at room temperature under reduced pressure to yield 90 mg of crystal A of (23S)-1α-hydroxy-27-nor-25-methylenevitamin D₃-26,23-lactone.

### <Example 7>

### Production of crystal A of (23S)-1α-hydroxy-27-nor-25-methylenevitamin D₃-26,23-lactone (crystallization from ethyl formate)

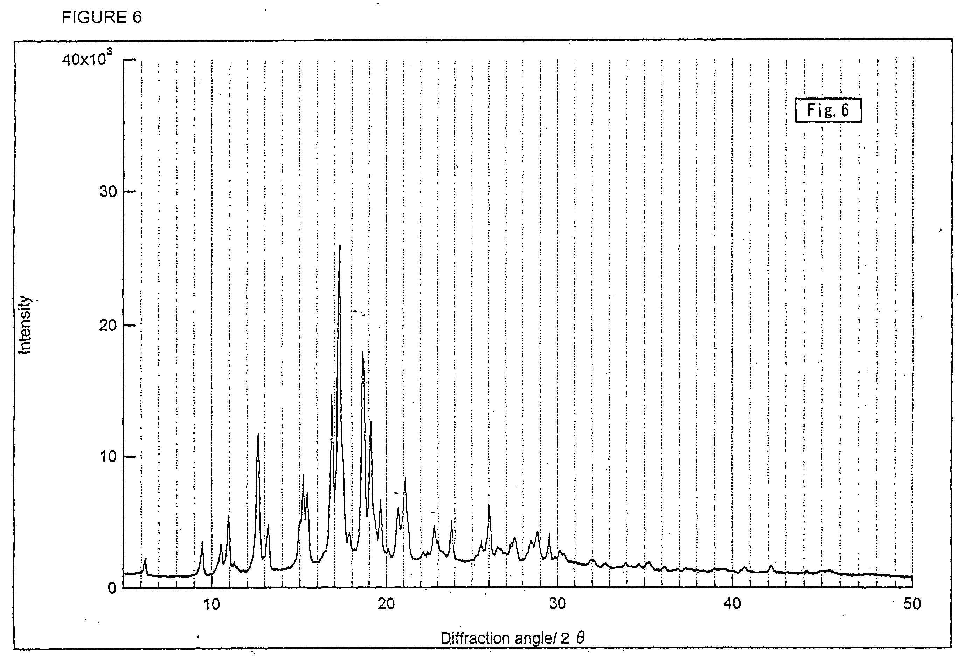

A 100 mg aliquot of (23S)-1α-hydroxy-27-nor-25-methylenevitamin D₃-26,23-lactone was dissolved in 100 µL of ethyl formate while warming. After cooling the solution to 25°C, the solution was left standing. After precipitation of crystals, the solution was further left standing at 20°C for 2 days. The precipitated crystals were filtered out and dried at room temperature under reduced pressure to yield 120 mg of crystal A of (23S)-1α-hydroxy-27-nor-25-methylenevitamin D₃-26,23-lactone. An XRD pattern of this crystal is shown in Fig. 6. The XRD measurement was performed in the similar manner as described in Reference example 1.

### <Example 8>

### Production of crystal A of (23S)-1α-hydroxy-27-nor-25-methylenevitamin D₃-26,23-lactone (crystallization from methyl acetate)

A 100 mg aliquot of (23S)-1α-hydroxy-27-nor-25-methylenevitamin D₃-26,23-lactone was dissolved in 100 µL of methyl acetate while warming. After cooling the solution to 25°C, the solution was left standing. After precipitation of crystals, the solution was further left standing at 20°C for 2 days. The precipitated crystals were filtered out and dried at room temperature under reduced pressure to yield 100 mg of crystal A of (23S)-1α-hydroxy-27-nor-25-methylenevitamin D₃-26,23-lactone.

### <Example 9>

### Production of crystal A of (23S)-1α-hydroxy-27-nor-25-methylenevitamin D₃-26,23-lactone (crystallization from ethyl acetate)

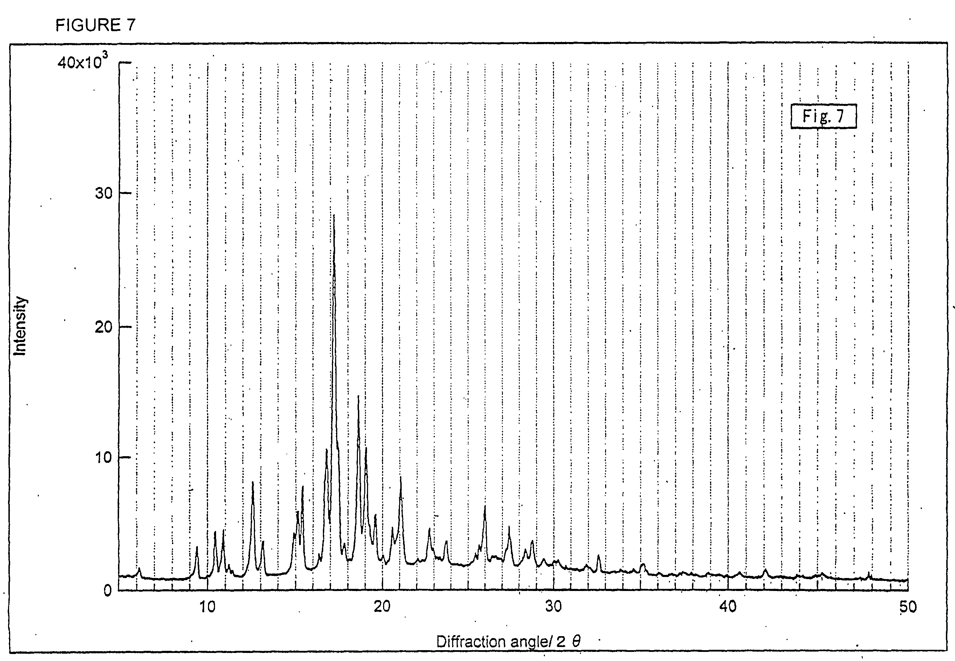

A 100 mg aliquot of (23S)-1α-hydroxy-27-nor-25-methylenevitamin D₃-26,23-lactone was dissolved in 150 µL of ethyl acetate while warming. After cooling the solution to 25°C, the solution was left standing. After precipitation of crystals, the solution was further left standing at 20°C for 2 days. The precipitated crystals were filtered out and dried at room temperature under reduced pressure to yield 114 mg of crystal A of (23S)-1α-hydroxy-27-nor-25-methylenevitamin D₃-26,23-lactone. An XRD pattern of this crystal is shown in Fig. 7. The XRD measurement was performed in the similar manner as described in Reference example 1.

### <Example 10>

### Production of crystal A of (23S)-1α-hydroxy-27-nor-25-methylenevitamin D₃-26,23-lactone (crystallization from isopropyl acetate)

A 100 mg aliquot of (23S)-1α-hydroxy-27-nor-25-methylenevitamin D₃-26,23-lactone was dissolved in 120 µL of isopropyl acetate while warming. After cooling the solution to 25°C, the solution was left standing. After precipitation of crystals, the solution was further left standing at 20°C for 2 days. The precipitated crystals were filtered out and dried at room temperature under reduced pressure to yield 110 mg of crystal A of (23S)-1α-hydroxy-27-nor-25-methylenevitamin D₃-26,23-lactone.

### <Example 11>

### Production of crystal A of (23S)-1α-hydroxy-27-nor-25-methylenevitamin D₃-26,23-lactone (crystallization from isobutyl acetate)

A 100 mg aliquot of (23S)-1α-hydroxy-27-nor-25-methylenevitamin D₃-26,23-lactone was dissolved in 150 µL of isobutyl acetate while warming. After cooling the solution to 25°C, the solution was left standing. After precipitation of crystals, the solution was further left standing at 20°C for 2 days. The precipitated crystals were filtered out and dried at room temperature under reduced pressure to yield 106 mg of crystal A of (23S)-1α-hydroxy-27-nor-25-methylenevitamin D₃-26,23-lactone.

### <Example 12>

### Stability tests of amorphous body of (23S)-1α-hydroxy-27-nor-25-methylenevitamin D₃-26,23-lactone and crystal A

The amorphous body obtained in Reference example 1, the sample of crystal A obtained in Example 1 (the sample crystallized from the mixed solvent of methanol and water), and the sample of crystal A obtained in Example 2 (the sample crystallized from the mixed solvent of ethanol and water) were stored in the dark at 60°C for 7 days. Theses samples were dissolved in ethanol, and the residue of each sample was quantitatively determined by using HPLC (A = 95% water/acetonitrile, B = 95% acetonitrile/water, B = 63%, detection at 265 nm). The results are shown in the following Table. Here, the "residue" refers to a relative amount (%) with respect to 100% of the initial sample.

| Sample | Residue (%) |
|---|---|
| Amorphous body (Reference Example 1) | 2.3 |
| Crystal A (Example 1, crystallized from the mixed solvent of methanol and water) | 89.8 |
| Crystal A (Example 2, crystallized from the mixed solvent of ethanol and water) | 87.2 |

From these results, it was found that the storage stability of the crystal of (23S)-1α-hydroxy-27-nor-25-methylenevitamin D₃-26,23-lactone of the present invention was dramatically improved as compared to that of the amorphous body. Therefore, since such a crystal is advantageous regarding the storage stability, manufacturing, process control, etc. of bulk drug substances and pharmaceutical compositions, it is useful in manufacturing pharmaceutical products.

### INDUSTRIAL APPLICABILITY

The crystal of (23S)-1α-hydroxy-27-nor-25-methylenevitamin D₃-26,23-lactone of the present invention is used as an active ingredient of pharmaceutical products. If the crystal of the present invention is used, it is advantageous in terms of the storage stability of pharmaceutical compositions as well as bulk drug substances. Also, it is advantageous in the process control of the manufacturing steps of them.

## Claims

1. A crystal of (23S)-1α-hydroxy-27-nor-25-methylenevitamin D₃-26,23-lactone which shows characteristic peaks of a powder X-ray diffraction pattern approximately at 12.65°, 15.05°, 15.20°, 16.85°, 17.30°, 17.50°, 18.70°, and 19.10°, whereby the reflection angle is expressed with 2θ.

2. A crystal of (23S)-1α-hydroxy-27-nor-25-methylenevitamin D₃-26,23-lactone which has characteristic peaks at 3482 cm⁻¹, 2946 cm⁻¹, 1744 cm⁻¹, 1663 cm⁻¹, 1433 cm⁻¹, 1364 cm⁻¹, 1281 cm⁻¹, 1144 cm⁻¹, 1038 cm⁻¹, 957 cm⁻¹, 897 cm⁻¹, 816 cm⁻¹, 741 cm⁻¹, 627 cm⁻¹, and 534 cm⁻¹ in an infrared spectroscopic analysis.

3. The crystal according to claim 1 or 2, which is obtained by crystallization from a solution that is prepared by dissolving (23S)-1α-hydroxy-27-nor-25-methylenevitamin D₃-26,23-lactone in an organic solvent.

4. The crystal according to claim 1 or 2, which is obtained by crystallization from a solution that is prepared by dissolving (23S)-1α-hydroxy-27-nor-25-methylenevitamin D₃-26,23-lactone in a mixed solvent consisting of an organic solvent and water.

5. The crystal according to claim 3 or 4, wherein the organic solvent is 1 kind or 2 kinds or more of organic solvents selected from the group consisting of methanol, ethanol, acetonitrile, methyl acetate, ethyl acetate, isopropyl acetate, isobutyl acetate, methyl formate, ethyl formate, and acetic acid.

6. A pharmaceutical composition that contains the crystal of (23S)-1α-hydroxy-27-nor-25-methylenevitamin D₃-26,23-lactone according to any one of claims 1 to 5 as an active ingredient.

7. The pharmaceutical composition according to claim 6, which is a therapeutic drug for osteoporosis, malignant tumor, psoriasis, hyperparathyroidism, inflammatory respiratory disease, rheumatoid arthritis, diabetes mellitus, hypertension, alopecia, acne, dermatitis, hypercalcemia, or Paget's disease of bone.

8. A process for producing the crystal according to claim 1 or 2, wherein the process is
**characterized by** crystallization from a solution that is prepared by dissolving (23S)-1α-hydroxy-27-nor-25-methylenevitamin D₃-26,23-lactone in an organic solvent.

9. A process for producing the crystal according to claim 1 or 2, wherein the process is
**characterized by** crystallization from a solution that is prepared by dissolving (23S)-1α-hydroxy-27-nor-25-methylenevitamin D₃-26,23-lactone in a mixed solvent consisting of an organic solvent and water.

10. The process for production according to claim 8 or 9, wherein the organic solvent e is 1 kind or 2 kinds or more of organic solvents selected from the group consisting of methanol, ethanol, acetonitrile, methyl acetate, ethyl acetate, isopropyl acetate, isobutyl acetate, methyl formate, ethyl formate, and acetic acid.

11. The process for production according to claim 8 or 9, wherein the organic solvent is 1 kind or 2 kinds or more of organic solvents selected from the group consisting of methanol, ethanol, acetonitrile, and acetic acid.

12. The process for production according to claim 8 or 9, wherein the organic solvent is acetonitrile.

13. The process for production according to claim 8 or 9, wherein the organic solvent is methanol.

14. The process for production according to claim 8 or 9, wherein the organic solvent is ethanol.
